# EUROPEAN PATENT SPECIFICATION

(11) **EP 2 155 647 B1**
(45) Date of publication and mention of the grant of the patent: **29.12.2010**
(21) Application number: 08759894.2
(22) Date of filing: 21.05.2008
(51) Int. Cl.: C07C 51/36, C07C 53/126

(54) **PROCESS FOR CONVERTING LEVULINIC ACID INTO PENTANOIC ACID**
VERFAHREN ZUR UMWANDLUNG VON LEVULINSÄURE IN PENTANSÄURE
PROCÉDÉ PERMETTANT DE CONVERTIR DE L'ACIDE LÉVULINIQUE EN ACIDE PENTANOÏQUE

(30) Priority: 22.05.2007 EP 07108621
(43) Date of publication of application: 24.02.2010
(73) Proprietor: Shell Internationale Research Maatschappij B.V., 2596 HR The Hague (NL)
(72) Inventor: AYOUB, Paul Marie, NL-1031 CM Amsterdam (NL); LANGE, Jean-Paul, NL-1031 CM Amsterdam (NL)
(74) Representative: Matthezing, Robert Maarten
(86) International application number: PCT/EP2008/056293
(87) International publication number: WO 2008/142127

(56) References cited:
- WO-A-2006/067171
- US-A1- 2006 100 449

## Description

### Field of the invention

The present invention provides a process for converting levulinic acid into pentanoic acid.

### Background of the invention

In WO2006/067171 is disclosed a process for the hydrogenation of levulinic acid to form pentanoic acid, wherein the levulinic acid is contacted, in the presence of hydrogen with a heterogeneous bi-functional catalyst, i.e. a strongly acidic heterogeneous catalyst having a hydrogenating component.

If levulinic acid is used as reactant in the process of WO2006/067171, catalyst deactivation might occur by leaching due to the presence of acid reactant and acid reaction product, by poisoning due to the presence of reaction water, and/or by fouling due to oligomerisation or polymerisation of unsaturated intermediates such angelica-lactone and pentenoic acid in the presence of an acid catalyst.

Since the hydrogenation of levulinic acid into pentanoic acid is highly exothermic, careful temperature control is very important to prevent undesired catalyst deactivation or side-reactions.

### Summary of the invention

It has now been found that levulinic acid can be catalytically hydrogenated to form pentanoic acid with limited catalyst deactivation and with minimum tar formation if the catalytic hydrogenation reaction is carried out in two separate steps with a non-acidic catalyst in the first step and an acidic and a hydrogenation catalytic function in the second step. Accordingly, the present invention provides a process for converting levulinic acid into pentanoic acid, the process comprising the following steps:
(a) contacting a feedstock comprising levulinic acid under hydrogenating conditions, in the presence of hydrogen, with a non-acidic heterogeneous hydrogenation catalyst comprising a hydrogenation metal supported on a solid catalyst carrier to obtain a first effluent comprising gamma valerolactone;
(b) contacting at least part of the first effluent under hydrogenating conditions, in the presence of hydrogen, with a strongly acidic catalyst and a hydrogenation metal to obtain a second effluent comprising pentanoic acid, wherein step (b) is operated at a conversion per pass of at most 70 wt%, to obtain the second effluent comprising pentanoic acid and unconverted gamma valerolactone; and wherein part of the unconverted gamma valerolactone is recycled to step (a) and/or step (b).

An advantage of the two-step process according to the invention as compared to the single-step process as disclosed in WO2006/067171 is that the heat released by the exothermic hydrogenation reaction can be better accommodated. Recycling of part of the effluent of step (a) and/or step (b), optionally in combination with effluent cooling, will provide for dilution of the acid concentration and, optionally, heat removal.

A further advantage is that there is less tar formation, since no acidic catalyst is present in step (a), i.e. the step wherein angelica-lactone is present.

### Brief description of the drawing

Figure 1 shows a first embodiment of the invention wherein steps (a) and (b) are carried out in a single adiabatically-operated stacked-bed reactor with a single cooled effluent recycle.
Figure 2 shows a second embodiment of the invention wherein steps (a) and (b) are carried out in two separated reactors with a cooled effluent recycle over each reactor.
Figures 3 and 4 show the hydrogenation of gamma-valero-lactone over a Pt/ZSM-5/SiO₂ under gas- and trickle phase operation.

### Detailed description of the invention

The process according to the invention comprises two steps. In step (a), levulinic acid is contacted under hydrogenating conditions with a non-acidic hydrogenation catalyst for conversion into gamma valerolactone. In step (b), at least part of the gamma valerolactone formed in step (a) is contacted under hydrogenating conditions with a strongly acidic catalyst and a hydrogenation metal to form pentanoic acid. The strongly acidic catalyst and the hydrogenation metal in step (b) may either be in the form of a bi-functional heterogeneous catalyst, i.e. a solid catalyst having both an acidic and a hydrogenation function, or in the form of a non-acidic solid hydrogenation catalyst and a liquid acidic catalyst.

In step (a), hydrogen and a feedstock comprising levulinic acid are brought into contact with the non-acidic catalyst under hydrogenating conditions. Without wishing to be bound to any theory, it is believed that gamma valerolactone is formed according to two different reaction paths. In the first path, levulinic acid is first hydrogenated to form 4-hydroxypentanoic acid and the hydroxy acid converted into gamma valerolactone by an internal (trans)esterification reaction. In the second reaction path, water is first eliminated to form angelica-lactone which is subsequently hydrogenated to form gamma valerolactone.

The hydrogen may be supplied to step (a) as pure hydrogen or as a hydrogen-containing gas. Hydrogen-containing gases suitable for hydrogenation reactions are well-known in the art.

The feedstock preferably comprises at least 50 wt% levulinic acid, more preferably at least 70 wt%, even more preferably at least 90 wt%. The feedstock comprising levulinic acid is contacted with the catalyst at hydrogenation conditions. Preferably step (a) is carried out at a temperature in the range of from 100 to 350 °C, more preferably of from 150 to 250 °C. The hydrogen pressure is preferably in the range of from 1 to 150 bar (absolute), more preferably of from 10 to 50 bar (absolute). The hydrogen to levulinic acid molar ratio is typically in the range of from 0.1 to 20. Preferably, an amount of hydrogen in excess of the stoichiometric amount is used in order to minimise the amount of the polymerising intermediate product alpha-angelicalactone. Therefore, the hydrogen to levulinic acid molar ratio is preferably in the range of from 1.1 to 5.0.

The levulinic acid and the reaction products of step (a), i.e. gamma valerolactone and water, may be in the liquid phase or in the gas phase during step (a), preferably in the liquid phase. The hydrogen is present in the gas phase. The reaction carried out in step (a) is therefore a gas phase reaction or a gas/liquid reaction in the presence of a solid catalyst. Step (a) may therefore be carried out in any reactor suitable for gas/solid reactions (in case of gaseous levulinic acid and gamma valerolactone) or for gas/liquid/solid reactions (in case of liquid levulinic acid and gamma valerolactone). If step (a) is carried out with the feedstock and reactants in the liquid phase, step (a) may for example be carried out in a trickle flow reactor comprising a fixed catalyst arrangement, a slurry bubble column or an ebullating bed reactor.

The catalyst in step (a) is a non-acidic hydrogenation catalyst comprising a hydrogenation metal supported on a solid catalyst carrier. The hydrogenation metal is preferably a metal of any one of column 7 to 11 of the Periodic table of Elements (latest IUPAC notation), more preferably a noble metal, even more preferably Ru, Rh, Pt, Pd, Ir and/or Au. The non-acidic solid catalyst carrier preferably is carbon or a non-acidic refractory oxide, more preferably silica, titania or zirconia. The catalyst carrier does not contain any acidic material such as zeolitic material or amorphous silica-alumina.

The concentration of the hydrogenating metal based on the total weight of the catalyst will typically be in the range of from 1 to 50 wt% for non-noble metals and in the range of from 0.01 to 5 wt% for noble metals. Preferred concentrations are from 2 to 20 wt% for non-noble metals and from 0.1 to 2 wt% for noble metals.

In order to minimise the concentration of acids and of polymerising compounds like alpha-angelicalactone in step (a), and thus avoid metal leaching of the catalyst and tar formation, step (a) is preferably carried out in a continuously stirred tank reactor, for example a slurry bubble column.

The first effluent comprises gamma valerolactone, water, hydrogen and optionally other reaction products such as methyltetrahydrofuran, pentanol, pentanediol, and unconverted levulinic acid.

In particular if step (a) is not carried out in a continuously stirred tank reactor, the acid concentration in step (a) may kept low by recycling part of the first effluent to step (a). In order to avoid adverse effects on the catalyst activity, preferably at least part of the water is removed from the first effluent before it is recycled to step (a). Water removal from the first effluent may be done by techniques known in the art, for example by flash separation or by distillation.

The conversion of levulinic acid into gamma valerolactone in step (a) is an exothermic reaction. Temperature control, in particular preventing the occurrence of hot spots, is therefore important. In particular in case of a fixed bed reactor, staged supply of the feedstock and/or staged supply of hydrogen is preferably applied in order to prevent hot spots. If part of the first effluent is recycled, the effluent is preferably cooled before being recycled in order to remove part of the heat released in the exothermic conversion step. Preferably, the recycled first effluent is cooled to a temperature in the range of from 20 to 200 °C, more preferably of from 40 to 100 °C before being recycled to step (a).

Preferably, step (a) is carried out in an adiabatically operated reactor with cooled effluent recycle. Alternatively, step (a) is carried out in an isothermally operated reactor by applying internal cooling.

In step (b), at least part of the first effluent is contacted, under hydrogenating conditions and in the presence of hydrogen, with a strongly acidic catalyst and a hydrogenation metal to obtain a second effluent comprising pentanoic acid.

Additional hydrogen may be supplied to step (b). If, however, the first effluent comprises sufficient hydrogen for the conversion of gamma valerolactone into pentanoic acid in step (b), no additional hydrogen needs to be supplied to step (b). Preferably, step (b) is operated at a conversion per pass of at most 50 wt%. Yet more preferably, the unconverted gamma valerolactone is separated from the second effluent, for example by distillation, before being recycled to step (a) and/or step (b).

The entire first effluent may be supplied to step (b) of the process according to the invention, in particular in case step (a) is being carried out in an CSTR and recycling of part of the first effluent to step (a) is not needed for heat removal.

Preferably, water is removed from the first effluent before it is supplied to step (b) in order to prevent adverse effects of water on the catalyst of step (b).

The second effluent comprises pentanoic acid, unconverted gamma valerolactone, hydrogen and optionally unconverted levulinic acid and water.

In step (b), the first effluent is contacted with a strongly acidic catalyst and a hydrogenation metal under hydrogenating conditions. Preferably, step (b) is carried out at a temperature in the range of from 150 to 350 °C, more preferably of from 200 to 300 °C, even more preferably of from 240 to 280 °C. The hydrogen pressure is preferably in the range of from 1 to 150 bar (absolute), more preferably of from 5 to 50 bar (absolute). In order to facilitate the flow of hydrogen from step (a) to step (b), the hydrogen pressure in step (b) is preferably lower than the hydrogen pressure in step (a), more preferably in the range of from 2 to 30 bar lower.

The hydrogen-to-gamma valerolactone molar ratio in step (b) is preferably in the range of from 0.1 to 10.

Preferably, the temperature in step (a) is lower than the temperature in step (b), more preferably in the range of from 30 to 100 °C lower.

In step (b), the reactant and the reaction product, i.e. gamma valerolactone and pentanoic acid, respectively, may be in the liquid phase or in the gas phase, preferably in the gas phase. Hydrogen is present in the gas phase. The reaction carried out in step (b) is therefore a gas phase reaction or a gas/liquid reaction in the presence of a solid catalyst. Step (b) may therefore be carried out in any reactor suitable for gas/solid reactions or gas/liquid/solid reactions. Preferably, step (b) is carried out at least in part under gas phase conditions.

Steps (a) and (b) may be carried out in a stacked bed configuration in a single reactor or in separate reactors. If carried out in a stacked bed configuration in a single reactor, the bed of step (a) is preferably operated at a lower temperature than the bed of step (b).

This may for example be achieved by applying a cooled recycle of the second effluent to step (a).

Step (b) is carried out in the presence of a strongly acidic catalytic function and a hydrogenation catalytic function. The first effluent is therefore contacted with both a strongly acidic catalyst and a hydrogenation metal. These functions are preferably combined in a bi-functional catalyst, i.e. an heterogeneous strongly acidic catalyst having a hydrogenation metal. In case of a heterogeneous strongly acidic catalyst having a hydrogenation metal, the catalyst preferably comprises an acidic zeolite, more preferably acidic zeolite beta or acidic ZSM-5, supporting at least one hydrogenation metal. Alternatively, such catalysts may comprise an acidic mixed oxide, sulphonated carbon, or temperature-resistant sulphonated resins.

Alternatively, the strongly acidic catalyst in step (b) is a homogeneous strongly acidic catalyst, for example a mineral acid or heteropolyacid such as tungstenphosphate or tungstensilicate, and the hydrogenation metal is supported on a solid non-acidic catalyst support, for example silica, titania or zirconia. Preferably, the liquid strongly acidic catalyst is a mineral acid, more preferably sulphuric acid or phosphoric acid, even more preferably sulphuric acid.

An advantage of using a liquid strongly acidic catalyst in combination with a hydrogenation metal on a solid non-acidic support in step (b) is that no strongly acidic catalyst support is needed, such as for example an acidic zeolite, and that leaching of such support due to the presence of the acid reaction product (pentanoic acid) is avoided.

The hydrogenation metal in the bi-functional catalyst or supported on the solid non-acidic catalyst support in step (b) is preferably a metal of any one of column 7 to 11 of the Periodic table of Elements, more preferably Ru, Rh, Pt, Pd, Ir and/or Au.

### Detailed description of the drawing

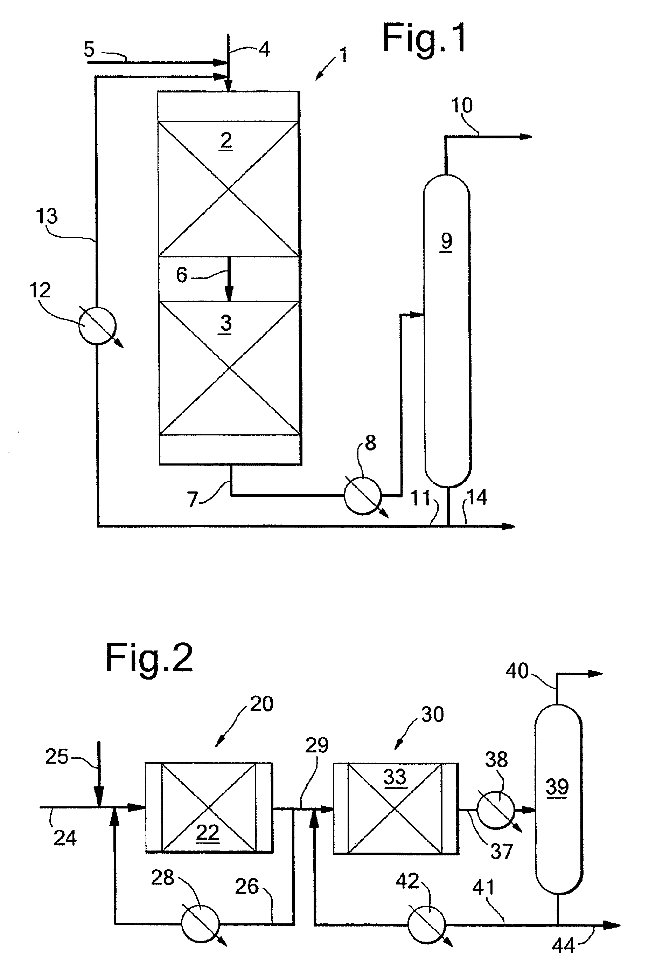

In Figure 1 is shown a reactor 1 comprising two catalyst beds (2, 3) in a stacked bed configuration. Catalyst bed 2 comprises a non-acidic heterogeneous hydrogenation catalyst and catalyst bed 3 comprises an acidic heterogeneous catalyst with a hydrogenation metal.

A feedstock comprising at least 90 wt% levulinic acid and hydrogen are supplied to reactor 1 via lines 4 and 5, respectively. The whole effluent 6 of catalyst bed 2 is fed to catalyst bed 3. The effluent of catalyst bed 3 is withdrawn from reactor 1 via line 7, cooled in cooler 8, and supplied to distillation column 9 for separation in a top stream comprising hydrogen, water and pentanoic acid and a bottoms stream mainly comprising gamma valerolactone. The top stream is withdrawn from column 9 via line 10 and the bottoms stream is withdrawn via line 11, cooled in cooler 12 and recycled to reactor 1 via line 13. Part of the bottoms stream may be purged via line 14.

Reactor 1 is adiabatically operated. The temperature is controlled by the use of a cooled gamma valerolactone recycle.

In Figure 2 is shown a line-up of the process according to the invention wherein steps (a) and (b) are carried out in two separated reactors 20, 30. Reactor 20 contains a catalyst bed 22 comprising a non-acidic heterogeneous hydrogenation catalyst. A feedstock comprising at least 90 wt% levulinic acid and hydrogen are supplied to reactor 20 via lines 24 and 25, respectively. Part of the effluent of catalyst bed 22 is recycled to reactor 20 via line 26 and cooler 28. The remainder of the effluent is supplied to reactor 30 via line 29. Reactor 30 contains a catalyst bed 33 comprising an acidic heterogeneous catalyst with a hydrogenation metal. The effluent of catalyst bed 33 is withdrawn from reactor 30 via line 37, cooled in cooler 38, and supplied to distillation column 39 for separation in a top stream comprising hydrogen, water and pentanoic acid and a bottoms stream mainly comprising gamma valerolactone. The top stream is withdrawn from column 39 via line 40 and the bottoms stream is withdrawn via line 41, cooled in cooler 42 and recycled to reactor 30 via line 43. Part of the bottoms stream may be purged via line 44. Both reactors 20 and 30 are adiabatically operated. The temperature is controlled by the use of cooled recycle streams.

### Example

The invention is now further illustrated by means of the following non-limiting example.

### Experiment 1

In this experiment, step (a) of the process according to the invention under iso-thermal conditions and with a relatively small recycle of the first effluent is illustrated.

A reactor tube with an internal diameter of 13 mm was loaded with a fixed bed of 6.0 grams of a heterogeneous catalyst comprising 0.8 wt% Pt on silica (cylindrical extrudates with a diameter of 1.6 mm) diluted with 6.3 grams silicon carbide particles and placed in an oven. The catalyst was reduced by pre-treating it with hydrogen (250 °C, 3h). The reactor was then brought to a temperature of 200 °C and a pressure of 40 bar gauge and a feedstock comprising 91.0 wt% levulinic acid, 7.8 wt% gamma valerolactone and 1.2 wt% water was supplied to the reactor at a weight hourly space velocity of 2.0 grams levulinic acid per gram catalyst per hour. Hydrogen was supplied to the reactor at a hydrogen-to-feedstock molar ratio of 4.5. These conditions were maintained for 500 hours. The effluent was collected and the composition determined by gas chromatography. In table 1, the concentration of levulinic acid (LA) and gamma valerolactone (GVL) in the effluent after certain times on stream (TOS) is given. The concentrations are expressed in mole% based on organic components on the effluent. Minor amounts of methyl-tetrahydrofuran (< 0.5 mole%), pentanoic acid (< 0.5 mole%) and unknown organic components (< 1.0 mole%) were found.

It can be seen from the results that the catalyst deactivates in time. Regeneration of the catalyst by calcining the catalyst in air at 450 °C for 24 hours resulted in restoration of its activity to its initial value.

**Table 1 Effluent composition experiment 1**

| TOS (h) | Feed/Effluent composition (mole%) | |
|---|---|---|
| | LA | GVL |
| 0 (feed) | 91 | 9 |
| 15 | 14 | 86 |
| 26 | 20 | 77 |
| 41 | 28 | 72 |
| 86 | 45 | 54 |
| 215 | 64 | 36 |
| 460 | 71 | 26 |

### Experiment 2

In this experiment, step (a) of the process according to the invention under iso-thermal conditions and with a relatively large recycle of gamma valerolactone is illustrated (dilution of levulinic acid feedstock with gamma valerolactone from first or second effluent, thereby simulating recycling of a relatively larger amount of the unreacted gamma valerolactone to step (a)).

A reactor tube with an internal diameter of 13 mm was loaded with a fixed bed of 6.0 grams of a heterogeneous catalyst comprising 0.8 wt% Pt on silica (cylindrical extrudates with a diameter of 1.6 mm) diluted with 6.3 grams silicon carbide particles and placed in an oven. The catalyst was reduced by pre-treating it with hydrogen (250 °C, 3h). The reactor was then brought to a temperature of 200 °C and a pressure of 40 bar gauge and a feedstock comprising 13 wt% levulinic acid and 87 wt% gamma valerolactone was supplied to the reactor at a weight hourly space velocity of 0.25 grams levulinic acid per gram catalyst per hour. Hydrogen was supplied to the reactor at a constant hydrogen flow of 1.7 litres (at STP) per gram catalyst per hour. The temperature, pressure and hydrogen flow conditions were maintained for 150 hours without catalyst reactivation. The weight hourly space velocity was increased to 0.5 g/g.h after 30 hours on stream, to 2.0 g/g.h after 123 hours on stream and decreased again to 0.25 after 127 hours on stream. The effluent was collected and the composition determined by gas chromatography. In table 2, the levulinic acid (LA) conversion (in mole% converted) and the concentration (in mole% of the effluent) of levulinic acid and gamma valerolactone (GVL) in the effluent at different times on stream (TOS) are given.

**Table 2. Experiment 2**

| TOS (h) | WHSV (g LA/g cat.) | LA conversion (mole%) | Effluent composition (mole%) | |
|---|---|---|---|---|
| | | | LA | GVL |
| 0 (feed) | | | 10 | 90 |
| 28 | 0.25 | 100 | 0 | 98 |
| 87 | 0.5 | 99 | 0.1 | 99 |
| 121 | 0.5 | 94 | 0.6 | 98 |
| 126 | 2.0 | 71 | 2.9 | 93 |
| 144 | 0.25 | 100 | 0 | 99 |

### Experiment 3

In this experiment, step (b) of the process according to the invention under iso-thermal conditions is illustrated.

A reactor tube with an internal diameter of 15 mm was loaded with a fixed bed of 17 grams of catalyst particles (cylindrical extrudates with a diameter of 1.6 mm) diluted with 30 grams silicon carbide particles. The catalyst contained 0.7 wt% Pt on an acidic carrier of 25 wt% surface-dealuminated ZSM-5 and 75 wt% silica binder.

The reactor tube was placed in an oven, reduced for 8 hours at 300 °C under a hydrogen flow of 30 litres (STP) per hour, pressured to a pressure of 45 bar (absolute) and brought to a temperature of 250 °C. A feedstock of pure gamma valerolactone was supplied to the reactor at a weight hourly space velocity of 1.0 gram per gram catalyst per hour. Hydrogen was fed to the reactor at a rate of 9.1 moles hydrogen per mole feedstock. Under these conditions the feedstock and the effluent were liquid. After 450 hours on stream, the temperature was raised to 275 °C and the pressure decreased to 10 bar (absolute). The feedstock and the effluent were in gaseous form at these conditions.

After 490 hours on stream, the experiment was continued at the initial conditions (250 °C, 45 bar).

The gamma valerolactone (GVL) conversion in mole% converted and the concentration of gamma-valerolactone (GVL), pentanoic acid (PA), methyl-tetrahydrofuran (MTHF), pentyl pentanoate (PP) and unknown organic compounds in mole% based on organic compounds in the effluent are given in table 3.

Apart from the components mentioned in table 3, pentane and 1-pentanol were found in the effluent in concentrations of 1-2 mole% and below 1 mole, respectively.

**Table 3. Experiment 3**

| TOS (h) | T (°C) | p (bar a) | GVL conversion (%mole) | Effluent composition (%mole) | | | | |
|---|---|---|---|---|---|---|---|---|
| | | | | GVL | PA | MTHF | PP | un-known |
| 5 | 250 | 45 | 67 | 33 | 54 | 0.8 | 3.2 | 6.5 |
| 25 | 250 | 45 | 50 | 50 | 33 | 2.0 | 2.6 | 6.7 |
| 35 | 250 | 45 | 42 | 58 | 30 | 2.8 | 2.5 | 2.8 |
| 113 | 250 | 45 | 32 | 68 | 17 | 6.9 | 2.0 | 2.3 |
| 285 | 250 | 45 | 25 | 75 | 9.1 | 8.5 | 1.3 | 2.4 |
| 429 | 250 | 45 | 21 | 80 | 8.6 | 5.9 | 1.0 | 3.1 |
| 460 | 275 | 10 | 35 | 65 | 21 | 2.0 | 2.2 | 3.4 |
| 480 | 275 | 10 | 38 | 62 | 26 | 1.8 | 2.3 | 3.1 |
| 502 | 250 | 45 | 23 | 77 | 11 | 5.8 | 1.4 | 3.2 |
| 620 | 250 | 45 | 16 | 84 | 5.3 | 5.0 | 0.4 | 3.4 |

Two further experiments (Experiments 4 and 5) were carried out to evaluate operating step (b) under gas-phase condition. In both experiments, the reaction was started under gas phase condition for 100-150 h and, then, abruptly switched to liquid phase condition by increasing the operating pressure (with simultaneous T change) to force the GVL partial pressure to exceed its condensation pressure.

### Experiment 4

The set-up and catalyst of examples 1 to 3 were employed, using a catalyst bed comprising 9.6 g of the catalyst diluted by 14.5 g of SiC.

The catalyst was reduced prior to the reaction under a hydrogen flow of 2500 Nl/kg/h at 10 bar with a temperature programmed to rise to 125°C in 5 h, then remaining at these conditions for 2 h, then increased further to 300°C within a period of 3.5 h, and finally stable at these conditions for 3 h before cooling down to reaction temperature.

Subsequently, the experiment was conducted for 270 h with GVL as feed. The run started under gas phase conditions at 275°C, 10 bar hydrogen pressure and a WHSV of 2 gGVL/gcat/h for 150 h and, subsequently, under trickle phase operation at 250°C, 45 bar hydrogen pressure and a WHSV = 1 g/g/h for about 100 h.

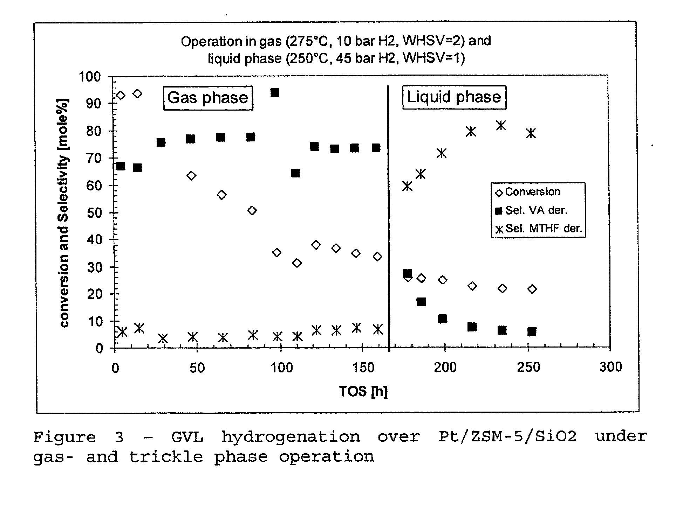

The catalyst activity declined throughout the run, in both gas and liquid phase. However, the selectivity for Valeric acid derivatives remained fairly stable around 75 mole% for 150 h under gas-phase condition but rapidly declined to <10 mol% after switching to trickle-phase condition while the selectivity for MTHF derivatives raised to 85 mole% (see Figure 3 and Table 4).

### Experiment 5

The set-up was as in example 4, and the catalyst bed consisted of 12 g of the same catalyst as employed in Example 4, diluted by 24 g of SiC. The catalyst was then reduced under the same conditions as shown in Example 4.

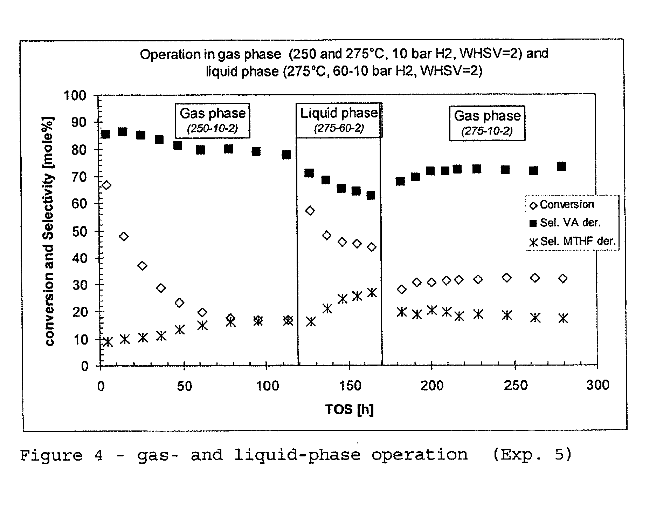

The experiment was then started under gas phase condition at 250°C, WHSV of 2 gGVL/gcat/h and 9 bar hydrogen pressure at a hydrogen/GVL molar ratio of 10 for 120 h, subsequently, under trickle phase operation at 275°C, 60 bar hydrogen pressure and a WHSV = 2 g/g/h for about 60 h and, then, back to gas phase condition at 275°C, 10 bar hydrogen pressure and a WHSV = 2 g/g/h for some 120 h. Operation in the gas-phase (250°C and 10 bar) resulted in high initial activity and selectivity to VA. The activity dropped monotonously throughout the run. However, selectivity remained high around 80% during the gas-phase operation. Upon switching to liquid-phase operation (275°C and 60 bar), the conversion was increased at once but both activity and selectivity declined with time. Switching back to the gas-phase by dropping the pressure back to 10 bar (275°C and 10 bar) resulted in an initial drop in conversion with partial recovery of the selectivity and subsequent stabilization of both activity and selectivity (Figure 4 and Table 5).

**Table 4 - results of Experiment 4**

| | Liquid phase | | | | | | | Gas Phase | | | | | |
|---|---|---|---|---|---|---|---|---|---|---|---|---|---|
| avg. Cat. Temp. (°C) | 275 | 275 | 275 | 275 | 275 | 275 | 275 | 250 | 250 | 250 | 250 | 250 | 250 |
| Press. (bar H₂) | 10 | 10 | 10 | 10 | 10 | 10 | 10 | 45 | 45 | 45 | 45 | 45 | 45 |
| 1/WHSV (g/g/h) | 0.8 | 0.5 | 0.5 | 0.4 | 0.5 | 0.5 | 0.5 | 1.0 | 1.0 | 1.0 | 1.0 | 1.0 | 1.0 |
| H2/feed (mol/mol) | 13.2 | 9.0 | 8.8 | 6.7 | 9.0 | 8.9 | 8.8 | 9.0 | 9.4 | 9.1 | 9.1 | 9.0 | 9.2 |
| TOS mid period (h) | 5.0 | 29.0 | 65.0 | 98.0 | 122.0 | 146.5 | 159.5 | 178.0 | 186.0 | 199.0 | 217.0 | 235.0 | 253.0 |
| | | | | | | | | | | | | | |
| GVL conversion | 92.9 | 75.5 | 56.3 | 35.0 | 37.8 | 34.7 | 33.4 | 25.7 | 25.5 | 24.7 | 22.5 | 21.5 | 21.5 |
| | | | | | | | | | | | | | |
| Selectivity | | | | | | | | | | | | | |
| Pentane (mol %) | 6 | 3 | 3 | 2 | 3 | 4 | 3 | 13 | 1.9 | 1.4 | 0.8 | 0.6 | 0.5 |
| MTHF (mol %) | 0 | 0 | 0 | 0 | 1 | 1 | 1 | 31 | 9.8 | 11.0 | 11.2 | 11.1 | 10.7 |
| 1-Pentanol (mol %) | 0 | 0 | 0 | 0 | 0 | 0 | 0 | 11 | 3.7 | 4.8 | 5.5 | 5.7 | 5.6 |
| Pentyl valerate (mol %) | 0 | 1 | 2 | 3 | 4 | 4 | 4 | 10 | 1.8 | 1.0 | 0.6 | 0.4 | 0.3 |
| Valeric Acid (mol %) | 67 | 75 | 76 | 92 | 72 | 71 | 71 | 22 | 3.3 | 2.0 | 1.3 | 1.1 | 1.0 |
| byproducts (mol %) | 5 | 5 | 4 | 6 | 4 | 4 | 5 | 7 | 2.0 | 2.4 | 2.1 | 2.0 | 2.1 |

**Table 5 - results of Exp. 5 (liquid phase in red)**

| | Gas Phase | | | | | Liquid Phase | | | Gas Phase | | | | |
|---|---|---|---|---|---|---|---|---|---|---|---|---|---|
| avg. Cat. Temp. (°C) | 250 | 250 | 250 | 250 | 250 | 273 | 275 | 275 | 275 | 275 | 275 | 275 | 275 |
| Press. (bar H2) | 10 | 10 | 10 | 10 | 10 | 60 | 60 | 60 | 10 | 10 | 11 | 10 | 10 |
| 1/WHSV (g/g/h) | 0.5 | 0.5 | 0.5 | 0.5 | 0.5 | 0.5 | 0.5 | 0.5 | 0.5 | 0.5 | 0.5 | 0.5 | 0.5 |
| H2/feed (mol/mol) | 9.1 | 9.0 | 9.0 | 9.0 | 9.1 | 9.2 | 9.5 | 9.4 | 9.0 | 8.9 | 8.8 | 9.2 | 8.9 |
| TOS mid period (h) | 5.0 | 25.5 | 47.5 | 78.5 | 113.0 | 127.0 | 146.5 | 164.5 | 182.5 | 191.5 | 209.5 | 228.5 | 279.5 |
| | | | | | | | | | | | | | |
| GVL conversion (100-GVL) | 66.9 | 37.0 | 23.4 | 17.7 | 17.0 | 57.3 | 45.6 | 43.8 | 28.0 | 30.6 | 31.5 | 31.8 | 31.9 |
| | | | | | | | | | | | | | |
| Sel. C4 (C1-C4 + C6) | 4.6 | 5.0 | 5.0 | 5.7 | 5.8 | 1.9 | 1.7 | 1.6 | 7.5 | 7.6 | 8.6 | 8.7 | 7.7 |
| Sel. C5 | 3.5 | 3.1 | 3.7 | 4.5 | 4.5 | 6.9 | 8.4 | 8.5 | 5.5 | 5.2 | 5.6 | 5.4 | 4.6 |
| Sel. MTHF | 0.2 | 1.1 | 3.0 | 4.3 | 4.5 | 3.9 | 9.8 | 11.7 | 3.6 | 3.0 | 2.4 | 2.1 | 2.0 |
| Sel. Pentanol | 0.1 | 0.1 | 0.3 | 0.4 | 0.5 | 0.4 | 1.0 | 1.2 | 0.9 | 0.7 | 0.6 | 0.5 | 0.5 |
| Sel. Pentyl Valerate | 1.3 | 2.3 | 2.8 | 2.9 | 2.8 | 6.1 | 7.3 | 7.4 | 4.9 | 4.8 | 5.0 | 4.8 | 4.6 |
| Sel. Valeric acid | 84.8 | 84.0 | 79.6 | 78.5 | 76.3 | 67.8 | 61.6 | 58.8 | 65.4 | 66.8 | 69.0 | 69.8 | 70.8 |
| Sel. byproducts | 1.1 | 1.1 | 2.0 | 2.4 | 2.9 | 1.4 | 2.2 | 2.2 | 3.4 | 3.9 | 3.0 | 2.9 | 2.8 |

## Claims

1. A process for converting levulinic acid into pentanoic acid, the process comprising the following steps:
(a) contacting a feedstock comprising levulinic acid under hydrogenating conditions, in the presence of hydrogen, with a non-acidic heterogeneous hydrogenation catalyst comprising a hydrogenation metal supported on a solid catalyst carrier to obtain a first effluent comprising gamma valerolactone;
(b) contacting at least part of the first effluent under hydrogenating conditions, in the presence of hydrogen, with a strongly acidic catalyst and a hydrogenation metal to obtain a second effluent comprising pentanoic acid, wherein step (b) is operated at a conversion per pass of at most 70 wt% to obtain the second effluent comprising pentanoic acid and unconverted gamma valerolactone, and wherein part of the unconverted gamma valerolactone is recycled to step (a) and/or step (b).

2. A process according to claim 1, wherein the entire first effluent is contacted with the strongly acidic catalyst and the hydrogenation metal in step (b).

3. A process according to claim 1, wherein part of the first effluent is recycled to step (a).

4. A process according to claim 3, wherein the part of the first effluent that is recycled is cooled before being recycled to step (a), preferably cooled to a temperature in the range of from 20 to 200 °C, more preferably 40 to 100 °C.

5. A process according to any one of the preceding claims, wherein step (a) is carried out in a continuously stirred tank reactor (CSTR).

6. A process according to any one of the preceding claims, wherein step (a) is carried out in a fixed bed reactor comprising a fixed bed of the non-acidic hydrogenation catalyst, the process comprising staged supply of levulinic acid to the fixed bed of the non-acidic hydrogenation catalyst.

7. A process according to any one of the preceding claims, wherein water is separated from the first effluent before the first effluent is contacted with the strongly acidic catalyst and the hydrogenation metal in step (b).

8. A process according to any one of the preceding claims, wherein the recycled unconverted gamma valerolactone is cooled before being supplied to step (a) and/or step (b).

9. A process according to any one of the preceding claims, wherein step (a) is carried out at a temperature in the range of from 150 to 250 °C.

10. A process according to any one of the preceding claims, wherein step (b) is carried out at a temperature in the range of from 200 to 300 °C, preferably of from 240 to 280 °C.

11. A process according to any one of the preceding claims, wherein the step (a) is carried out at a lower temperature than step (b), preferably in the range of from 30 to 100 °C lower.

12. A process according to any one of the preceding claims, wherein the strongly acidic catalyst and the hydrogenation metal in step (b) are combined in a heterogeneous strongly acidic catalyst having a hydrogenation metal.

13. A process according to any one of claims 1 to 12, wherein the strongly acidic catalyst in step (b) is a liquid strongly acidic catalyst and the hydrogenation metal is supported on a solid non-acidic catalyst support.

14. A process according to any one of claims 1 to 12, wherein step (b) is carried out at least in part under gas phase conditions.

## Patentansprüche

1. Verfahren zum Umwandeln von Levulinsäure in Pentansäure, wobei das Verfahren die folgenden Schritte umfasst:
(a) In-Kontakt-Bringen einer Charge, welche Levulinsäure umfasst, mit einem nicht sauren heterogenen Hydrierungskatalysator, welcher ein Hydriermetall umfasst, das auf einem festen Katalysatorträger getragen wird, unter Hydrierbedingungen in Gegenwart von Wasserstoff, um einen ersten Abfluss zu erhalten, welcher γ-Valerolacton umfasst;
(b) In-Kontakt-Bringen zumindest eines Teils des ersten Abflusses mit einem stark sauren Katalysator und einem Hydriermetall unter Hydrierbedingungen in Gegenwart von Wasserstoff, um einen zweiten Abfluss zu erhalten, welcher Pentansäure umfasst, wobei der Schritt (b) mit einem Umsatz je Durchlauf von höchstens 70 Gew.-% durchgeführt wird, um den zweiten Abfluss zu erhalten, welcher Pentansäure und nicht umgesetztes γ-Valerolacton umfasst, und wobei ein Teil des nicht umgesetzten Y-Valerolactons in das Verfahren des Schrittes (a) und/oder des Schrittes (b) zurückgeführt wird.

2. Verfahren nach Anspruch 1, wobei im Schritt (b) der vollständige erste Abfluss mit dem stark sauren Katalysator und dem Hydriermetall in Kontakt gebracht wird.

3. Verfahren nach Anspruch 1, wobei ein Teil des ersten Abflusses in das Verfahren des Schrittes (a) zurückgeführt wird.

4. Verfahren nach Anspruch 3, wobei der Teil des ersten Abflusses, der zurückgeführt wird, gekühlt wird, vorzugsweise auf eine Temperatur im Bereich von 20 °C bis 200 °C, insbesondere 40 °C bis 100 °C, bevor er zu Schritt (a) zurückgeführt wird.

5. Verfahren nach einem der vorhergehenden Ansprüche, wobei der Schritt (a) in einem Durchflussrührkessel-Reaktor (Continuously Stirred Tank Reaktor, CSTR) durchgeführt wird.

6. Verfahren nach einem der vorhergehenden Ansprüche, wobei der Schritt (a) in einem Festbettreaktor durchgeführt wird, welcher ein Festbett des nicht sauren Hydrierungskatalysators umfasst, wobei das Verfahren eine gestufte Zufuhr der Levulinsäure in das Festbett des nicht sauren Hydrierungskatalysators umfasst.

7. Verfahren nach einem der vorhergehenden Ansprüche, wobei aus dem ersten Abfluss Wasser abgetrennt wird, bevor der erste Abfluss im Schritt (b) mit dem stark sauren Katalysator und dem Hydriermetall in Kontakt gebracht wird.

8. Verfahren nach einem der vorhergehenden Ansprüche, wobei das zurückgeführte nicht umgesetzte γ-Valerolacton gekühlt. wird, bevor es Schritt (a) und/oder Schritt (b) zugeführt wird.

9. Verfahren nach einem der vorhergehenden Ansprüche, wobei Schritt (a) bei einer Temperatur im Bereich von 150 °C bis 250 °C durchgeführt wird.

10. Verfahren nach einem der vorhergehenden Ansprüche, wobei Schritt (b) bei einer Temperatur im Bereich von 200 °C bis 300 °C, vorzugsweise 240 °C bis 280 °C, durchgeführt wird.

11. Verfahren nach einem der vorhergehenden Ansprüche, wobei Schritt (a) bei einer niedrigeren Temperatur als Schritt (b) durchgeführt wird, vorzugsweise bei einer um 30 °C bis 100 °C niedrigeren Temperatur.

12. Verfahren nach einem der vorhergehenden Ansprüche, wobei der stark saure Katalysator und das Hydriermetall im Schritt (b) in einem heterogenen stark sauren Katalysator, welcher ein Hydriermetall aufweist, kombiniert sind.

13. Verfahren nach einem der Ansprüche 1 bis 12, wobei es sich bei dem stark sauren Katalysator im Schritt (b) um einen flüssigen stark sauren Katalysator handelt und das Hydriermetall auf einem festen nicht sauren Katalysatorträger getragen wird.

14. Verfahren nach einem der Ansprüche 1 bis 12, wobei Schritt (b) zumindest teilweise unter Gasphasenbedingungen durchgeführt wird.

## Revendications

1. Processus de conversion d'acide lévulinique en acide pentanoïque, le processus comprenant les étapes suivantes :
(a) mettre en contact un produit de départ comprenant de l'acide lévulinique dans des conditions d'hydrogénation, en présence d'hydrogène, avec un catalyseur d'hydrogénation hétérogène non-acide comprenant un métal d'hydrogénation soutenu sur un support de catalyseur solide afin d'obtenir un premier effluent comprenant du gamma-valérolactone ;
(b) mettre en contact au moins une partie du premier effluent dans des conditions d'hydrogénation, en présence d'hydrogène, avec un catalyseur fortement acide et un métal d'hydrogénation afin d'obtenir un deuxième effluent comprenant de l'acide pentanoïque, dans lequel l'étape (b) est mise en oeuvre avec un taux de conversion par passe de 70 % en poids maximum afin d'obtenir le deuxième effluent comprenant de l'acide pentanoïque et du gamma-valérolactone non converti, et dans lequel une partie du gamma-valérolactone non converti est recyclée à l'étape (a) et/ou à l'étape (b).

2. Processus selon la revendication 1, dans lequel l'ensemble du premier effluent est mis en contact avec le catalyseur fortement acide et le métal d'hydrogénation à l'étape (b).

3. Processus selon la revendication 1, dans lequel une partie du premier effluent est recyclée à l'étape (a).

4. Processus selon la revendication 3, dans lequel la partie du premier effluent qui est recyclée est refroidie avant d'être recyclée à l'étape (a), elle est de préférence refroidie à une température comprise entre 20 et 200°C, de manière plus préférable entre 40 et 100°C.

5. Processus selon l'une quelconque des revendications précédentes, dans lequel l'étape (a) est réalisée dans un réacteur à fonctionnement continu (CSTR - Continuously stirred tank reactor).

6. Processus selon l'une quelconque des revendications précédentes, dans lequel l'étape (a) est réalisée dans un réacteur à lit fixe comprenant un lit fixe du catalyseur d'hydrogénation non-acide, le processus comprenant une fourniture par étapes d'acide lévulinique au lit fixe du catalyseur d'hydrogénation non-acide.

7. Processus selon l'une quelconque des revendications précédentes, dans lequel l'eau est séparée du premier effluent avant que le premier effluent vienne au contact du catalyseur fortement acide et du métal d'hydrogénation à l'étape (b).

8. Processus selon l'une quelconque des revendications précédentes, dans lequel le gamma-valérolactone non converti recyclé est refroidi avant d'être fourni à l'étape (a) et/ou à l'étape (b).

9. Processus selon l'une quelconque des revendications précédentes, dans lequel l'étape (a) est réalisée à une température comprise entre 150 et 250°C.

10. Processus selon l'une quelconque des revendications précédentes, dans lequel l'étape (b) est réalisée à une température comprise entre 200 et 300°C, de préférence entre 240 et 280°C.

11. Processus selon l'une quelconque des revendications précédentes, dans lequel l'étape (a) est réalisée à une température inférieure à celle de l'étape (b), de préférence dans une plage inférieure comprise entre 30 et 100°C.

12. Processus selon l'une quelconque des revendications précédentes, dans lequel le catalyseur fortement acide et le métal d'hydrogénation à l'étape (b) sont combinés dans un catalyseur fortement acide hétérogène comprenant un métal d'hydrogénation.

13. Processus selon l'une quelconque des revendications 1 à 12, dans lequel le catalyseur fortement acide à l'étape (b) est un catalyseur liquide fortement acide et le métal d'hydrogénation est soutenu sur un support de catalyseur solide non-acide.

14. Processus selon l'une quelconque des revendications 1 à 12, dans lequel l'étape (b) est réalisée au moins en partie dans des conditions de phase gazeuse.
